# EUROPÄISCHE PATENTSCHRIFT

(11) **EP 3 123 168 B1**
(45) Veröffentlichungstag und Bekanntmachung des Hinweises auf die Patenterteilung: **04.04.2018**
(21) Anmeldenummer: 15736191.6
(22) Anmeldetag: 11.06.2015
(51) Int. Cl.: G01N 33/46, G01N 1/08, B26F 1/38

(54) **HOLZPRÜFWERKZEUG UND VERFAHREN ZUM SICHTPRÜFEN EINES HOLZOBJEKTS**
WOOD TEST TOOL AND METHOD FOR VISUALLY CHECKING A WOOD OBJECT
OUTIL DE CONTRÔLE DU BOIS ET PROCÉDÉ DE CONTRÔLE VISUEL D'UN OBJET EN BOIS

(30) Priorität: 15.10.2014 DE 102014015051
(43) Veröffentlichungstag der Anmeldung: 01.02.2017
(73) Patentinhaber: IML-Instrumenta Mechanik Labor GmbH, 69168 Wiesloch (DE)
(72) Erfinder: HUNGER, Erich, 76133 Karlsruhe (DE); HUNGER, Sebastian, 69181 Leimen (DE); HUNGER, Fabian, 69181 Leimen (DE)
(74) Vertreter: mepat Patentanwälte
(86) Internationale Anmeldenummer: PCT/EP2015/001173
(87) Internationale Veröffentlichungsnummer: WO 2016/058650

(56) Entgegenhaltungen:
- WO-A1-84/01827
- Anonymous: "BGS Locheisensatz, 565, 9-tlg. in Kunststoffkassette, 3-12 mm: Amazon.de: Baumarkt", , 25. April 2007 (2007-04-25), XP055216610, Gefunden im Internet: URL:http://www.amazon.de/BGS-Locheisensatz -9-tlg-Kunststoffkassette-3-12/dp/B000PTQ9 YM/ref=pd_sim_60_1/276-4580283-3687517?ie= UTF8&refRID=0BB2CKQ24V7HKVB0ZZRJ&dpID=41%2 Bws9DGGoL&dpSrc=sims&preST=_AC_UL160_SR160 %2C160_ [gefunden am 2015-09-28]
- KOTTLORS C ET AL: "Probenahme aus Holz and Holzwerkstoffen zur Klärung von Fragen des Holzschutzes durch chemische Untersuchungen", HOLZ ALS ROH- UND WERKSTOFF, SPRINGER-VERLAG. BERLIN, DE, Bd. 51, Nr. 2, 3. August 2013 (2013-08-03) , Seiten 126-134, XP035180488, ISSN: 0018-3768, DOI: 10.1007/BF03325376 [gefunden am 2013-08-03]
- FURNISS M M: "A Circular Punch for Cutting Samples of Bark Infested with Beetles", CANADIAN ENTOMOLOGIST, OTTAWA, CA, Bd. 94, Nr. 9, September 1962 (1962-09), Seiten 959-963, XP008177618, ISSN: 0008-347X, DOI: 10.4039/ENT94959-9 [gefunden am 2012-05-31]

## Beschreibung

Die Erfindung betrifft ein Holzprüfwerkzeug zum Ausstanzen und Sichtprüfen eines Holzprobenkerns. Zudem betrifft die Erfindung ein Verfahren zur Überprüfung der Tränktiefe eines imprägnierten Holzobjekts.

Bäume, aber auch Holzmasten und andere der Witterung ausgesetzte Holzobjekte müssen von Zeit zu Zeit untersucht werden, ob ihre Standfestigkeit noch gewährleistet ist, oder ob im Inneren des Holzobjekts Fäulnis etc. vorliegt. Hierzu werden Bohrwiderstandsmessungen vorgenommen, wobei aus dem gemessenen Bohrwiderstand Rückschlüsse auf den Zustand des Holzes gezogen werden. Sind die zu untersuchenden Holzobjekte imprägniert, so hat dies Einfluss auf die Bohrwiderstandsmessung. Daher ist es wichtig, im Vorfeld zu wissen, wie tief eine Imprägnierung reicht, etwa um entsprechende Einstellungen bei der Auswertung der Messergebnisse vornehmen zu können.

So ist die Ermittlung der Tränktiefe ergänzend zur Bohrwiderstandsmessung ein wichtiger Parameter zur Beurteilung der Masten hinsichtlich Fäulnis. Häufig treten Fäulnisprobleme bei Masten wegen schlechter Imprägnierung auf. Mit Hilfe der Tränktiefe kann beginnende Fäule und können bestehende Hohlräume, die durch die Bohrwiderstandsmessung festgestellt werden, besser und aussagekräftiger bewertet werden.

Weiter sind aus dem Stand der Technik Locheisen bekannt, die zum Herausstanzen einer Probe aus einem Werkstück eingesetzt werden und einen hohlzylindrischen Stift aufweisen, der einenends eine mit dem Innendurchmesser des Hohlzylinders korrespondierende Eintriebsöffnung und anderenends einen abgeflachten Kopf und ein Sichtfenster aufweist.

Ausgehend von diesem Stand der Technik ist es Aufgabe der vorliegenden Erfindung, ein Werkzeug zu schaffen, mit dem einfach die Tränktiefe eines Holzobjekts nachgewiesen werden kann.

Diese Aufgabe wird durch ein Holzprüfwerkzeug mit den Merkmalen des Anspruchs 1 und durch das Holzprüfset mit den Merkmalen des Anspruchs 8 gelöst.

Weiterbildungen sind in den Unteransprüchen ausgeführt.

Ein Verfahren mit den Merkmalen des Anspruchs 9 ermöglicht in einfacher und schneller Weise die Überprüfung der Tränktiefe eines Holzobjekts.

Ein erfindungsgemäßes Holzprüfwerkzeug dient zum Ausstanzen und Sichtprüfen eines Holzprobenkerns, um die Tränk- bzw. Imprägniertiefe eines imprägnierten Holzobjekts zu überprüfen. Dazu weist das Holzprüfwerkzeug einen hohlzylindrischen Stift auf, der einenends eine mit dem Innendurchmesser des Hohlzylinders korrespondierende Eintriebsöffnung und anderenends einen abgeflachten Kopf aufweist. Weiter hat der hohlzylindrische Stift ein Sichtfenster in Form einer Längsausnehmung, die sich, ausgehend von dem eintriebsöffnungsseitigen Ende des hohlzylindrischen Stifts über zumindest zwei Drittel entlang der Länge des Hohlzylinders erstreckt und so schnell und einfach nach dem Ausstanzen eine optische Kontrolle des in dem Holzylinder aufgenommenen Holzprobenkerns gestattet.

Das Sichtfenster kann sich gegebenenfalls bis zum Kopf erstrecken, so dass das Holzprüfwerkzeug maximal in das zu prüfende Holzobjekt eingetrieben und damit die Länge des durch das Sichtfenster überprüfbaren Holzprobenkerns maximiert werden kann. Die Breite des Sichtfensters ist dabei so konzipiert, dass der Holzprobenkern gut erkannt werden kann und gleichzeitig eine ausreichende Stabilität des Holzwerkzeugs zum Eintreiben in das Holzobjekt gewährleistet ist. Daher ist vorgesehen, dass die Breite des Sichtfensters höchstens ein Viertel, bevorzugt näherungsweise ein Achtel oder weniger des Außenumfangs des hohlzylindrischen Stifts ausmacht.

Für eine besonders einfache und schnelle Evaluierung der Eindringtiefe des hohlzylindrischen Stifts im zu prüfenden Holzobjekt und der Tränktiefe kann bei einer weiteren erfindungsgemäßen Ausführungsform des Werkzeugs an der äußeren Mantelfläche des hohlzylindrischen Stifts eine Skala in Längsrichtung beispielsweise benachbart zu dem Sichtfernster oder alternativ umlaufend vorgesehen sein, die sich zumindest entlang der Länge des Sichtfensters erstreckt. Dabei bildet die Eintriebsöffnung einen Bezugspunkt der Skala, unabhängig davon, ob sich die Skala bis zu der Eintriebsöffnung erstreckt oder nicht, so dass die Eindringtiefe des Holzprüfwerkzeugs mit der Skala korreliert ist.

Damit das Holzprüfwerkzeug gut in das zu prüfende Holzobjekt eingetrieben werden kann, kann das Ende mit der Eintriebsöffnung verjüngt sein und/oder eine Schneidkante aufweisen. Der zum Eintreiben erforderliche Kraftaufwand kann dadurch verringert werden.

Um die zum Eintreiben erforderliche Stabilität und eine dauerhafte Haltbarkeit aufzuweisen, kann das Holzprüfwerkzeug aus Metall, bevorzugt Stahl oder einem anderen Material mit ausreichender Härte - gegebenenfalls ist auch ein Kunststoff mit hoher Festigkeit, Schlagzähigkeit, Steifigkeit und Härte wie z. B. ein Polycarbonat oder Polyphenylensulfid denkbar - gefertigt sein.

Um den Holzprobenkern innerhalb des Holzprüfwerkzeugs zu fixieren, so dass der Holzprobenkern zusammen gehalten wird und ein korrektes Ablesen an der Skala möglich ist, kann das Holzprüfwerkzeug einen elastisch komprimierbaren Zylinderstopfen aufweisen, der in dem hohlzylindrischen Stift kopfendenseitig angeordnet ist. Durch den Zylinderstopfen wird insbesondere bei einem Werkzeug mit durchgängiger Bohrung verhindert, dass schon beim Eintreiben des Werkzeugs Stücke des Probenkerns aus der Öffnung am Kopf austreten können. Der Durchmesser des Zylinderstopfens entspricht dem Innendurchmesser des Hohlzylinders und seine Länge entspricht zumindest der Hälfte der Länge des Hohlzylinders. Vorzugsweise ist der Zylinderstopfen aus einem Schaumstoff gefertigt; es sind aber auch andere elastisch verformbare Materialien, wie etwa Gummi, oder federnde Konstruktionen denkbar.

Um die Entnahme des Holzprüfwerkzeugs nach dem Eintreiben aus dem Holzobjekt zu erleichtern, kann der Kopf einen größeren Durchmesser aufweisen als der Stift, so dass der Kopf durch ein entsprechendes Werkzeug entsprechend einem Nagelheber aus dem Holzobjekt gezogen werden kann. Bevorzugt kann der Kopf dabei konusartig ausgeformt sein, wodurch ein Überstand des Kopfes beim Eintreiben einfacher einzuhalten ist, was das Hintergreifen mit dem Nagelheber vereinfacht.

Ein erfindungsgemäßes Verfahren zur Überprüfung der Tränktiefe eines imprägnierten Holzobjekts verwendet ein erfindungsgemäßes Holzprüfwerkzeug und umfasst das Eintreiben des Holzprüfwerkzeugs mit der Eintriebsöffnung in das Holzobjekt, wobei ein Holzprobenkern in dem hohlzylindrischen Stift aufgenommen wird. Für die jeweiligen Holzobjekte liegen bekannte Werte für die Tränktiefe vor, die üblicherweise im Bereich von 2 bis 4 cm liegen, gegebenenfalls aber auch, etwa im Falle von Kiefernholz, bei 8 bis 10 cm liegen können. Die Tiefe, mit der der Stift in das Holzobjekt eingetrieben wird, wird daher entsprechend größer gewählt, beispielsweise mehr als 5 cm bzw. zwischen 5 und 10 cm oder aber auch über 10 cm. Nachdem das Holzprüfwerkzeug aus dem Holzobjekt wieder herausgezogen wurde, erfolgt durch das Sichtfenster des Holzprüfwerkzeugs das Sichtprüfen des Holzprobenkerns, der zumindest einen durch Imprägnierung verfärbten Abschnitt und einen nicht verfärbten Abschnitt aufweist. Anhand der Länge des verfärbten Abschnitts, der vorzugsweise mittels der an dem Holzprüfwerkzeug vorliegenden Skala abgelesen werden kann, kann die Tränktiefe erfasst werden.

Ferner ist es vorteilhaft, zum Entfernen des Holzprobenkerns und insofern zum Sichten einer Vielzahl Kerne nacheinander ein Holzprüfset zu verwenden, das das erfindungsgemäße Holzprüfwerkzeug, einen Ausstoßer und eine Führungshülse umfasst. Diese weist eine Bohrung auf, die dazu ausgebildet ist, den Hohlzylinder passend aufzunehmen, und die sich einenends zu einem Aufnahmeabschnitt für die Spitze des Holzprüfwerkzeugs mit der Eintriebsöffnung aufweitet, so dass das Holzprüfwerkzeug mit der Eintriebsöffnung an der Spitze in dem Aufnahmeabschnitt der Führungshülse aufgenommen werden kann. Der Ausstoßer wird dann vom anderen Ende der Führungshülse her durch die Bohrung zentriert in den hohlzylindrischen Stift eingeführt und hat daher entsprechende, auf den Hohlzylinder abgestimmte Abmessungen. Durch Schieben des Ausstoßers mit leichtem Druck kann so der Holzprobenkern entnommen werden und das Werkzeug steht für die nächste Probennahme zur Verfügung.

Weitere Ausführungsformen sowie einige der Vorteile, die mit diesen und weiteren Ausführungsformen verbunden sind, werden durch die nachfolgende ausführliche Beschreibung unter Bezug auf die begleitenden Figuren deutlich und besser verständlich. Gegenstände oder Teile derselben, die im Wesentlichen gleich oder ähnlich sind, können mit denselben Bezugszeichen versehen sein. Die Figuren sind lediglich eine schematische Darstellung einer Ausführungsform der Erfindung.

Dabei zeigen:
- **Fig. 1**: eine perspektivische Ansicht eines erfindungsgemäßen Holzprüfwerkzeugs,
- **Fig. 2**: eine perspektivische Ansicht eines erfindungsgemäßen Holzprüfwerkzeugs mit Probenkern,
- **Fig. 3**: a) eine Querschnittansicht durch einen mit metallverstärkter Folie geschützten Holzmast und b) ein entsprechender Stanzkern,
- **Fig. 4**: a) eine Querschnittansicht durch einen Holzmast mit geringerer Tränktiefe und b) ein entsprechender Stanzkern,
- **Fig. 5**: eine perspektivische Ansicht eines Betätigungswerkzeugs beim Herausziehen eines erfindungsgemäßen Holzprüfwerkzeugs aus einem Holzobjekt,
- **Fig. 6**: eine perspektivische Seitenansicht eines Holzprüfwerkzeugs mit einem Set zum Entfernen des Probenkerns.

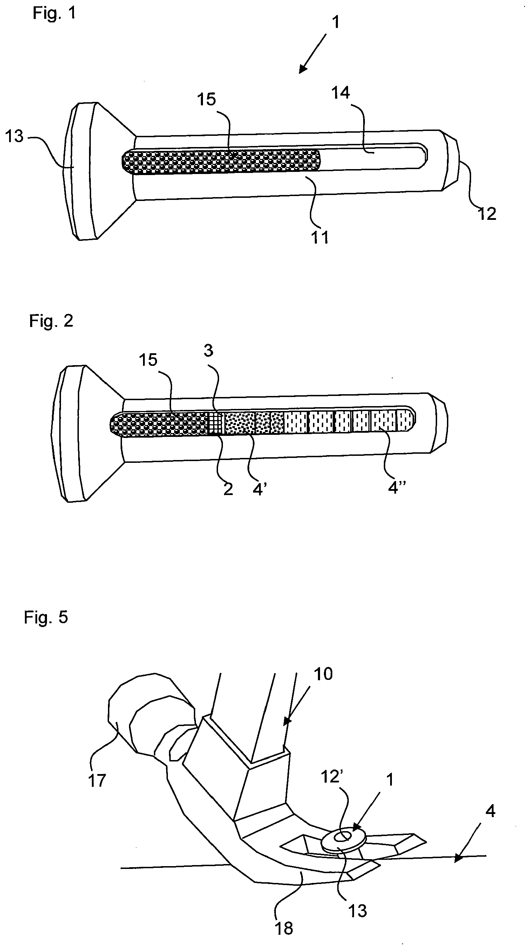

Mit dem erfindungsgemäßen Holzprüfwerkzeug 1, das in **Fig. 1** dargestellt ist, kann die Tränktiefe imprägnierter Holzobjekte, wie z. B. Holzmasten, überprüft werden.

Das Holzprüfwerkzeug 1 besteht aus einem hohlzylindrischen Stift 11, der einenends eine Eintriebsöffnung 12 aufweist, die mit dem Innendurchmesser des Hohlzylinders korrespondiert. Anderenends hat der hohlzylindrische Stift 11 einen abgeflachten Kopf 13. Gegebenenfalls kann die Bohrung durch den Stift 11 auch durchgehend sein, so dass auch am Kopf 13 eine Öffnung 12' vorliegen kann, wie in **Fig. 5** angedeutet ist. Der hohlzylindrische Stift 11 eines erfindungsgemäßen Holzprüfwerkzeugs 1 kann beispielsweise eine Länge von 10 cm aufweisen, um so Eindringtiefen von bis etwa 8 cm gut realisieren zu können, und damit Tränktiefen von bis zu 7 cm gut erkennen zu können. Der Innendurchmesser des hohlzylindrischen Stifts 11 kann dann beispielsweise 4 bis 5 mm betragen. Diese Werte sind allerdings nur beispielhaft zu verstehen, Abweichungen davon sind im Schutzumfang der Erfindung enthalten.

Der im hohlzylindrischen Stift 11 angeordnete elastisch komprimierbare Zylinderstopfen 15, der vorzugsweise aus Schaumstoff besteht, fixiert dann nicht nur den Holzprobenkern im Stift 11, sondern verschließt auch die zweite Öffnung 12' und verhindert so, dass der Probenkern dort austreten kann. Das Sichtfenster 14 in dem hohlzylindrischen Stift 11 ermöglicht, den Holzprobenkern direkt nach dem Ausstanzen beurteilen zu können, ohne diesen aus dem Holzprüfwerkzeug 1 entfernen zu müssen, wie dies in **Fig. 2** gezeigt ist. Damit ist gesichert, dass der Kern intakt bleibt.

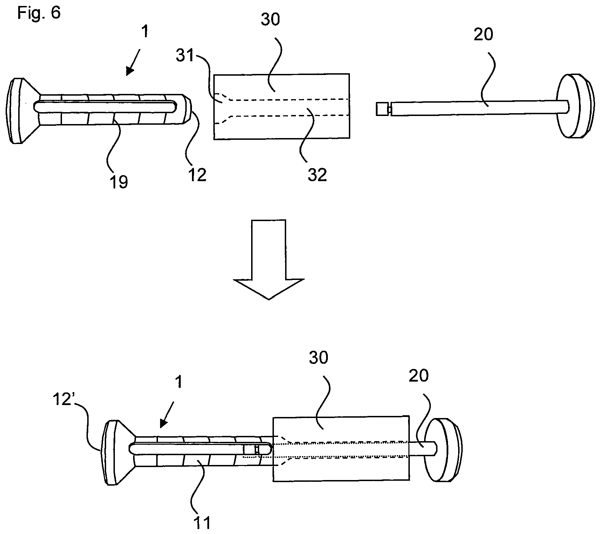

Der Zylinderstopfen 15 aus Schaumstoff wird beim Ausstanzen des Holzprobenkerns, der die durch Imprägnierung verfärbten Abschnitte 4' und die unverfärbten nicht-imprägnierten Abschnitte 4" aufweist, komprimiert, wodurch der Holzprobenkern im Inneren des Stifts 11 gehalten wird, auch wenn er trocken und bröselig ist. So kann die Tränktiefe leicht erfasst werden, insbesondere wenn neben dem Sichtfenster 14 eine Skala angeordnet (z. B. aufgedruckt oder eingelasert) ist. Eine Skala mit umlaufend eingebrachten Kerben 19, die den Abstand zur Werkzeugspitze mit der Eintriebsöffnung 12 markieren, ist in **Fig. 6** skizziert. Mit einer solchen Skala kann die Eindringtiefe des Holzprüfwerkzeugs 1 beim Eintreiben in ein Holzobjekt kontrolliert werden.

Die Länge des Zylinderstopfens 15 ist beliebig, solange er die Aufgaben erfüllt, den Holzprobenkern im Stift zu halten. Vorzugsweise kann der Zylinderstopfen 15 eine Länge entsprechend dem Stift 11 aufweisen, so dass er mit dem Anlegen des Holzprüfwerkzeugs 1 an dem Holzobjekt anliegt, und dann beim Eintreiben des Holzprüfwerkzeugs 1 in das Holzobjekt durch den eindringenden Holzprobenkern zusammengedrückt wird. Weist das Holzprüfwerkzeug 1 eine Kopföffnung 12' auf, wie in Fig. 5 skizziert ist, kann der Zylinderstopfen 15 je nach Länge beim Eintreiben des Holzprüfwerkzeugs 1 in das Holzobjekt durch den eindringenden Holzprobenkern auch etwas aus der Kopföffnung 12' herausgedrückt werden. Dies wirkt sich allerdings nicht nachteilig aus, der Holzprobenkern wird nach wie vor in dem Hohlzylinder gehalten, so dass die Tränktiefe anhand eines zusammengehaltenen Probenkerns beurteilt werden kann.

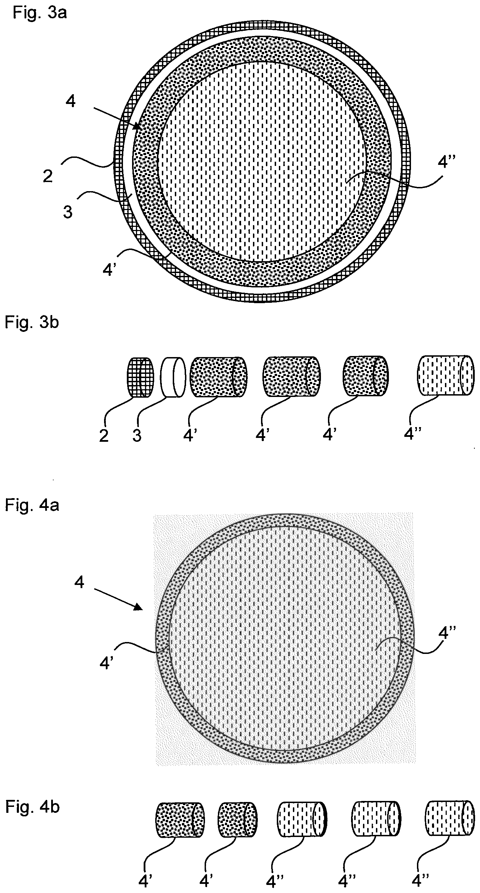

**Fig. 3a** zeigt einen mit einer zweilagigen Folie aus Metall 3 und Kunststoff 2 vor mechanischen und biologischen Beschädigungen geschützten Mast 4 mit einem imprägnierten Abschnitt 4' und einem nicht imprägnierten Abschnitt 4" als zu prüfendes Holzobjekt.

**Fig. 3b** verdeutlich, wie in diesem Fall der Holzprobenkern aussieht: Neben den imprägnierten Abschnitten 4' und dem nicht imprägnierten Abschnitt 4", der sich farblich von den imprägnierten Abschnitten 4' unterscheidet, umfasst der Probenkern dann auch noch ein ausgestanztes Metall- und Folienstück 3,2. Ein entsprechender Probenkern ist auch in **Fig. 2** in dem Holzprüfwerkzeug 1 durch das Sichtfenster 14 zu sehen. Die Darstellung des Holzprobenkerns in mehreren Abschnitten ist der Tatsache geschuldet, dass ein solcher Holzprobenkern in der Realität in Abschnitte zerfallen kann.

**Fig. 4a** zeigt einen nicht geschützten Holzmast, bei dem der imprägnierte Abschnitt 4' eine geringere Tiefe aufweist als der in **Fig. 3a****.** Dies zeigt sich auch in dem in **Fig. 4b** dargestellten Holzprobenkern mit den imprägnierten Abschnitten 4' und den nicht imprägnierten Abschnitten 4".

Um auch Proben aus Holzobjekten 4, die wie der Holzmast aus **Fig. 3a** mit einer folienumwickelten Metallschicht (z. B. Messing) geschützt sind, mit dem erfindungsgemäßen Holzprüfwerkzeug 1 entnehmen zu können, ist dieses aus gehärtetem Stahl gefertigt. Für weichere Hölzer können gegebenenfalls auch Holzprüfwerkzeuge 1 aus anderen Stahl- oder Metalllegierungen, gegebenenfalls auch Kunststoff, eingesetzt werden.

Das Eindringen in das Holzobjekt wird unterstützt, indem das eintriebsöffnungsseitige Ende des hohlzylindrischen Stifts 11 zugespitzt bzw. verjüngt ist. Alternativ oder zusätzlich kann die Eintriebsöffnung mit Schneidkante ausgeführt sein.

Der Kopf 13 weist üblicherweise einen größeren Durchmesser auf als der Stift 11, um einfacher aus dem Holzobjekt 4 herausgezogen werden zu können, wie in **Fig. 5** zu sehen ist. Das hierzu verwendete Werkzeug 10 entspricht einem Zimmermannshammer, der einen muldenförmigen Teil mit Keilschlitz, den so genannten Nagelheber 18, und einen Hammerkopf 17 aufweist, der vorteilhaft zuvor zum Eintreiben des Holzprüfwerkzeugs 1 in das Holzobjekt zum Einsatz gekommen ist.

Um einen Probenkern einfach und definiert aus dem hohlzylindrischen Stift 11 entfernen zu können, kann ein Ausstoßerset, wie in **Fig. 6** beispielhaft gezeigt, zum Einsatz kommen, das einen Ausstoßer 20 und eine Führungshülse 30 umfasst. Die Führungshülse 30 hat eine Bohrung 32, die sich einenends zu einem Aufnahmeabschnitt 31 für die Spitze des Holzprüfwerkzeugs 1 mit der Eintriebsöffnung 12 aufweitet. Zum Ausstoßen des Probenkerns wird das Holzprüfwerkzeug 1 mit der Eintriebsöffnung 12 an der Spitze in dem Aufnahmeabschnitt 31 der Führungshülse 30 platziert und der Ausstoßer 20 vom anderen Ende der Führungshülse 30 her durch die Bohrung 32 und so zentriert in den hohlzylindrischen Stift 11 eingeführt, um den dort vorliegenden Probenkern aus der Kopföffnung 12' auszustoßen.

## Patentansprüche

1. Holzprüfwerkzeug (1) zum Ausstanzen und Sichtprüfen eines Holzprobenkerns, wobei das Holzprüfwerkzeug (1) einen hohlzylindrischen Stift (11) aufweist, der einenends eine mit dem Innendurchmesser des Hohlzylinders korrespondierende Eintriebsöffnung (12) und anderenends einen abgeflachten Kopf (13) und ein Sichtfenster (14) aufweist, das sich ausgehend von dem eintriebsöffnungsseitigen Ende des hohlzylindrischen Stifts (11) über zumindest zwei Drittel entlang der Länge des Hohlzylinders erstreckt, **dadurch gekennzeichnet, dass** das Sichtfenster eine Breite aufweist, die höchstens ein Viertel des Außenumfangs des hohlzylindrischen Stifts (11) ausmacht.

2. Holzprüfwerkzeug (1) nach Anspruch 1,
**dadurch gekennzeichnet, dass**
das Sichtfenster (14) sich bis zum Kopf (13) erstreckt und/oder eine Breite aufweist, die höchstens ein Achtel des Außenumfangs des hohlzylindrischen Stifts (11) ausmacht.

3. Holzprüfwerkzeug (1) nach Anspruch 1 oder 2,
**dadurch gekennzeichnet, dass**
an der äußeren Mantelfläche des hohlzylindrischen Stifts (11) eine Skala in Längsrichtung benachbart zu dem Sichtfenster (14) oder umlaufend vorgesehen ist, die sich zumindest entlang der Länge des Sichtfensters (14) erstreckt, wobei die Eintriebsöffnung (12) einen Bezugspunkt der Skala bildet.

4. Holzprüfwerkzeug (1) nach zumindest einem der Ansprüche 1 bis 3,
**dadurch gekennzeichnet, dass**
das Ende mit der Eintriebsöffnung (12) verjüngt ist und/oder eine Schneidkante aufweist.

5. Holzprüfwerkzeug (1) nach zumindest einem der Ansprüche 1 bis 4,
**dadurch gekennzeichnet, dass**
das Holzprüfwerkzeug (1) aus Metall, bevorzugt Stahl, gefertigt ist.

6. Holzprüfwerkzeug (1) nach zumindest einem der Ansprüche 1 bis 5,
**dadurch gekennzeichnet, dass**
das Holzprüfwerkzeug (1) einen elastisch komprimierbaren Zylinderstopfen (15) aufweist, der in dem hohlzylindrischen Stift (11) kopfendenseitig angeordnet ist und dessen Durchmesser dem Innendurchmesser des Hohlzylinders entspricht, wobei der Zylinderstopfen (15) eine Länge aufweist, die zumindest der Hälfte der Länge des Hohlzylinders entspricht.

7. Holzprüfwerkzeug (1) nach zumindest einem der Ansprüche 1 bis 3,
**dadurch gekennzeichnet, dass**
der Kopf (13) einen größeren Durchmesser aufweist als der Stift (11), und bevorzugt konusartig ausgeformt ist.

8. Holzprüfset, das
- ein Holzprüfwerkzeug (1) nach zumindest einem der Ansprüche 1 bis 7,
- einen Ausstoßer (20) und
- eine Führungshülse (30) umfasst, wobei
die Führungshülse (30) eine Bohrung (32) aufweist, die sich einenends zu einem Aufnahmeabschnitt (31) für die Spitze des Holzprüfwerkzeugs (1) mit der Eintriebsöffnung (12) aufweitet, und
wobei das Holzprüfwerkzeug (1) mit der Eintriebsöffnung (12) an der Spitze in dem Aufnahmeabschnitt (31) der Führungshülse (30) aufnehmbar ist und
der Ausstoßer (20) vom anderen Ende der Führungshülse (30) her durch die Bohrung (32) zentriert in den hohlzylindrischen Stift (11) einführbar ist.

9. Verfahren zur Überprüfung der Tränktiefe eines imprägnierten Holzobjekts (4) unter Verwendung eines Werkzeugs (1) nach zumindest einem der Ansprüche 1 bis 7 oder des Holzprüfsets nach Anspruch 8,
**umfassend die Schritte:**
- Eintreiben des Holzprüfwerkzeugs (1) mit der Eintriebsöffnung (12) in das Holzobjekt (4), wobei ein Holzprobenkern in dem hohlzylindrischen Stift (11) aufgenommen wird, bis zu einer Eindringtiefe, die größer ist als eine vermutete Tränktiefe,
- Herausziehen des Holzprüfwerkzeugs (1) aus dem Holzobjekt (4),
- durch das Sichtfenster (14) des Holzprüfwerkzeugs (1) Sichtprüfen des Holzprobenkerns, der zumindest einen durch Imprägnierung verfärbten Abschnitt (4') und einen nicht verfärbten Abschnitt (4") aufweist, und Erfassen der Tränktiefe anhand einer Länge des verfärbten Abschnitts (4').

10. Verfahren nach Anspruch 9,
**umfassend die Schritte:**
Einführen des Holzprüfwerkzeugs (1) mit der Eintriebsöffnung (12) an der Spitze in den Aufnahmeabschnitt (31) der Führungshülse (30),
- zentriert Einführen des Ausstoßers (20) vom anderen Ende der Führungshülse (30) her durch die Bohrung (32) in den hohlzylindrischen Stift (11),
durch Druck auf den Ausstoßer (20) Ausstoßen des Holzprobenkerns aus der Kopföffnung (12').

## Claims

1. A wood test tool (1) for punching out and visually checking a wood sample core, wherein the wood test tool (1) has a hollow cylindrical pin (11), which at one end has an input opening (12), which corresponds to the inner diameter of the hollow cylinder, and at the other end has a flattened head (13) and a viewing window (14), which, starting from the end of the hollow cylindrical pin (11) on the side of the input opening, extends across at least two thirds along the length of the hollow cylinder,
**characterized in that**
the viewing window has a width, which amounts to not more than a quarter of the outer circumference of the hollow cylindrical pin (11).

2. The wood test tool (1) according to claim 1,
**characterized in that**
the viewing window (14) extends as far as the head (13) and/or has a width, which amounts to not more than one eighth of the outer circumference of the hollow cylindrical pin (11).

3. The wood test tool (1) according to claim 1 or 2,
**characterized in that**
a scale is provided on the outer jacket surface of the hollow cylindrical pin (11) in the longitudinal direction adjacent to the viewing window (14) or circumferentially, which scale extends at least along the length of the viewing window (14), wherein the input opening (12) forms a reference point of the scale.

4. The wood test tool (1) according to at least any one of claims 1 to 3,
**charact rized in that**
the end with the input opening (12) is tapered and/or has a cutting edge.

5. The wood test tool (1) according to at least any one of claims 1 to 4,
**characterized in that**
the wood test tool (1) is made of metal, preferably of steel.

6. The wood test tool (1) according to at least any one of claims 1 to 5,
**characterized in that**
the wood test tool (1) has an elastically compressible cylinder plug (15), which is arranged in the hollow cylindrical pin (11) at the top end, and the diameter of which corresponds to the inner diameter of the hollow cylinder, wherein the cylinder plug (15) has a length, which corresponds to at least half of the length of the hollow cylinder.

7. The wood test tool (1) according to at least any one of claims 1 to 3,
**characterized in that**
the head (13) has a larger diameter than the pin (11), and is preferably shaped in a cone-like manner.

8. A wood test set, which comprises
- a wood test tool (1) according to at least any one of claims 1 to 7,
- an ejector (20) and
- a guide sleeve (30), wherein
the guide sleeve (30) has a hole (32), which widens at the one end towards a receiving section (31) for the top of the wood test tool (1) with the input opening (12), and
wherein the wood test tool (1) with the input opening (12) at the top can be received in the receiving section (31) of the guide sleeve (30) and the ejector (20) can be inserted into the hollow cylindrical pin (11) from the other end of the guide sleeve (30) through the hole (32) in a centred manner.

9. A method for checking the impregnation depth of an impregnated wood object (4) using a tool (1) according to at least any one of claims 1 to 7 or the wood test set according to claim 8,
**comprising the steps:**
- driving the wood test tool (1) with the input opening (12) into the wood object (4), wherein a wood sample core is received in the hollow cylindrical pin (11) up to a penetration depth, which is larger than an assumed impregnation depth,
- pulling the wood test tool (1) out of the wood object (4),
- visually checking the wood test core, which hast at least one section (4') discoloured by impregnation and a non-discoloured section (4"), through the viewing window (14) of the wood test tool (1), and determining the impregnation depth by means of a length of the discoloured section (4').

10. The method according to claim 9,
**comprising the steps:**
inserting the wood test tool (1) with the input opening (12) at the top into the receiving section (31) of the guide sleeve (30),
- inserting the ejector (20) in a centred manner into the hollow cylindrical pin (11) from the other end of the guide sleeve (30) through the hole (32),
ejecting the wood sample core from the head opening (12') by applying pressure to the ejector (20).

## Revendications

1. Outil de contrôle du bois (1) pour la découpe à la presse et le contrôle visuel d'une âme d'échantillon de bois, dans lequel l'outil de contrôle du bois (1) présente une tige (11) cylindrique creuse qui présente à une extrémité, une ouverture d'introduction (12) correspondant au diamètre interne du cylindre creux et à l'autre extrémité, une tête aplatie (13) et une fenêtre de regard (14) qui s'étend sur au moins deux tiers sur la longueur du cylindre creux en partant de l'extrémité côté ouverture d'introduction de la tige (11) cylindrique creuse,
**caractérisé en ce que**
la fenêtre de regard présente une largeur qui fait au plus un quart du périmètre extérieur de la tige (11) cylindrique creuse.

2. Outil de contrôle du bois (1) selon la revendication 1,
**caractérisé en ce que** la fenêtre de regard (14) s'étend jusqu'à la tête (13) et/ou présente une largeur qui fait au plus un huitième du périmètre extérieur de la tige (11) cylindrique creuse.

3. Outil de contrôle du bois (1) selon la revendication 1 ou 2,
**caractérisé en ce qu'une** échelle dans le sens longitudinal voisine de la fenêtre de regard (14) ou circulaire est prévue sur la surface d'enveloppe extérieure de la tige (11) cylindrique creuse, qui s'étend au moins le long de la longueur de la fenêtre de regard (14), dans lequel l'ouverture d'introduction (12) forme un point de référence de l'échelle.

4. Outil de contrôle du bois (1) selon au moins l'une des revendications 1 à 3,
**caractérisé en ce que** l'extrémité avec l'ouverture d'introduction (12) est rétrécie et/ou présente une arête de coupe.

5. Outil de contrôle du bois (1) selon au moins l'une des revendications 1 à 4,
**caractérisé en ce que** l'outil de contrôle du bois (1) est fabriqué en métal, de préférence en acier.

6. Outil de contrôle du bois (1) selon au moins l'une des revendications 1 à 5,
**caractérisé en ce que** l'outil de contrôle du bois (1) présente un bouchon de cylindre (15) pouvant être comprimé élastiquement, qui est agencé côté extrémité de tête dans la tige (11) cylindrique creuse et dont le diamètre correspond au diamètre interne du cylindre creux, dans lequel le bouchon de cylindre (15) présente une longueur qui correspond au moins à la moitié de la longueur du cylindre creux.

7. Outil de contrôle du bois (1) selon au moins l'une des revendications 1 à 3,
**caractérisé en ce que** la tête (13) présente un plus grand diamètre que la tige (11) et est conique de préférence.

8. Kit de contrôle du bois qui comprend
- un outil de contrôle du bois (1) selon au moins l'une des revendications 1 à 7,
- un éjecteur (20) et
- un manchon de guidage (30), dans lequel
le manchon de guidage (30) présente un alésage (32) qui s'élargit à une extrémité vers un tronçon de réception (31) pour la pointe de l'outil de contrôle du bois (1) avec l'ouverture d'introduction (12), et
dans lequel l'outil de contrôle du bois (1) avec l'ouverture d'introduction (12) à la pointe peut être reçu dans le tronçon de réception (31) du manchon de guidage (30) et
l'éjecteur (20) peut être introduit dans la tige (11) cylindrique creuse en étant centré à travers l'alésage (32), de l'autre extrémité du manchon de guidage (30).

9. Procédé de contrôle d'une profondeur d'imprégnation d'un objet en bois (4) imprégné par l'emploi d'un outil (1) selon au moins l'une des revendications 1 à 7 ou du kit de contrôle du bois selon la revendication 8,
**comprenant les étapes :**
- d'introduction de l'outil de contrôle du bois (1) avec l'ouverture d'introduction (12) dans l'objet en bois (4), dans lequel une âme d'échantillon de bois est reçue dans la tige (11) cylindrique creuse, jusqu'à une profondeur de pénétration qui est supérieure à une profonde d'imprégnation supposée,
- retrait de l'outil de contrôle du bois (1) hors de l'objet en bois (4),
- à travers la fenêtre de regard (14) de l'outil de contrôle du bois (1), contrôle visuel de l'âme d'échantillon de bois, qui présente au moins un tronçon coloré (4') par imprégnation et un tronçon non coloré (4"), et détection de la profondeur d'imprégnation à l'aide d'une longueur du tronçon (4') coloré.

10. Procédé selon la revendication 9,
**comprenant les étapes :**
- d'introduction de l'outil de contrôle du bois (1) avec l'ouverture d'introduction (12) à la pointe dans le tronçon de réception (31) du manchon de guidage (30),
- d'introduction centrée de l'éjecteur (20) dans la tige (11) cylindrique creuse à travers l'alésage (32) de l'autre extrémité du manchon de guidage (30), par pression sur l'éjecteur (20),
- éjection de l'âme d'échantillon de bois hors de l'ouverture de tête (12').
